# EUROPEAN PATENT SPECIFICATION

(11) **EP 0 412 592 B1**
(45) Date of publication and mention of the grant of the patent: **21.06.1995**
(21) Application number: 90201965.2
(22) Date of filing: 18.07.1990
(51) Int. Cl.: C07D 405/06

(54) **Nitrofurantoin crystals**
Nitrofurantoinkristalle
Cristaux de nitrofurantoine

(30) Priority: 26.07.1989 US 386050
(43) Date of publication of application: 13.02.1991
(73) Proprietor: Norwich Eaton Pharmaceuticals, Inc., Norwich New York 13815 (US)
(72) Inventor: Cazer, Frederick Dana, Earlville, NY 13332 (US); Scott, Barry Lee, Norwich, NY 13815 (US); Kane, Michael John, Norwich, NY 13815 (US); Shahi, Vijay, Lucknow, U.P. (ID)
(74) Representative: Brooks, Maxim Courtney

(56) References cited:
- CHEMICAL ABSTRACTS, vol. 67, no. 13, September 25, 1967 Columbus, Ohio, USAHENRY E. PAUL et al. "Laboratory studies with nitrofurantoin. Relationship between crystal size, urinary excretion in the rat and man, and emesis indogs" page 5892, column 1, abstract no. 62 880d
- CHEMICAL ABSTRACTS, vol. 75, no. 11, September 13, 1971 Columbus, Ohio,USA BIAVATI, GIANNI et al. "Pharmacokinetic study of nitrofurantoin using macrocrystals with differentiated granulometry" page 276, column 1, abstract no74 373u
- CHEMICAL ABSTRACTS, vol. 89, no. 12, September 18, 1978 Columbus, Ohio,USA MANNISTO P. "The effect of crystal size, gastric content and emptying rate on the absorption of nitrofurantoin in healthy human volunteers" page 301,column 1, abstract no. 94 919u
- CHEMICAL ABSTRACTS, vol. 111, no. 24, Dezember 11, 1989 Columbus, Ohio,USA MARSHALL, P.V. et al. "Crystallization solvent induced solid-state andparticulate modifications of nitrofurantoin" page 313, column 1, abstract no.219 217v
- CHEMICAL ABSTRACTS, vol. 110, no. 6, February 6, 1989 Columbus, Ohio,USA DELGADO A. et al. "Electrophoretic study on nonstabilized nitrofurantoin suspensions" page 378, column 2, abstract no. 44 826t
- CHEMICAL ABSTRACTS, vol. 86, no. 6, February 7, 1977 Columbus, Ohio, USA BORSA,M. et al. "Nitrofurantoin: crystal dimensions and bioavailability" page 291,column 1, abstract no. 34 195h

## Description

### BACKGROUND OF THE INVENTION

This invention relates to materials useful as antibacterial agents in humans and other animals, and compositions containing such materials. In particular, it relates to a novel crystalline form of nitrofurantoin.

Nitrofurantoin is an antibacterial agent used extensively in the treatment of urinary tract infections. It is rapidly absorbed in the gastrointestinal tract, and reaches high concentrations in the urine. A general description of nitrofurantoin is found in D. E. Cadwallader, 15 J. American Pharmaceutical Association 409 (1975); and J. D. Conklin, "The Pharmacokinetics of Nitrofurantoin and Its Related Bioavailability," 25 Antibiotics and Chemotherapy 233 (1978).

As with many other pharmaceutical active materials, the pharmacokinetics of nitrofurantoin may be affected by the size and type of nitrofurantoin crystals used in a dosage form. See, for example, J. K. Haleblian, "Characterization of Habits and Crystalline Modification of Solids and Their Pharmaceutical Applications", 66 J. Pharmaceutical Sciences 1269 (1975). In particular, the use of macrocrystalline nitrofurantoin has been found to reduce the level of emetic side effects that may be associated with nitrofurantoin. This association is discussed in the following articles: H. E. Paul et al., "Laboratory Studies with Nitrofurantoin", 56 J. Pharmaceutical Sciences 882 (1967), and N. Garti et al., "Habit Modifications of Nitrofurantoin Crystallized from Formic Acid Mixtures", 6 Drug Development and Industrial Pharmacy 379 (1980). Macrocrystalline nitrofurantoin having a surface area of from 120 cm² /g (0.012 m²/g) to 1000 cm²/g (0.1 m²/g) is described in U.S. Patent 3,401,221, Borgmann et al., issued September 10, 1968.

Nitrofurantoin is marketed by Norwich Eaton Pharmaceuticals, Inc., in several dosage forms using nitrofurantoin of differing crystal size. One such dosage form is a suspension of nitrofurantoin monohydrate. The crystals of nitrofurantoin in these suspensions are fine particles typically smaller than 170 mesh. Another solid dosage form contains relatively large crystals of anhydrous nitrofurantoin (from 40 to 200 [from 420 »m to 74 »m] mesh), in a capsule (marketed under the tradename "Macrodantin"). The BET surface area of these macrocrystals is from 0.06 m²/g to 0.15 m²/g.

A variety of dosage forms of nitrofurantoin are also known in the pharmaceutical literature. For example, solid oral dosage forms of nitrofurantoin are described in U.S. Patent 4,122,157, Huber, issued October 24, 1978; U.S. Patent 4,370,313, Davies, issued January 25, 1983; European Patent Publication 250,023, Patel et al., published December 23, 1987; and European Patent Publication 250,038, Patel, published December 23, 1987. Suspensions of nitrofurantoin are described in N. Shah et al., "Effect of Polymers on Dissolution from Drug Suspensions", 65 J. Pharmaceutical Sciences 1618 (1976).

None of these references, however, describes an aqueous suspension of macrocrystalline nitrofurantoin. Such a suspension would combine the highly desirable pharmacokinetics of commercially available macrocrystalline nitrofurantoin capsules (Macrodantin), with the benefits of pharmaceutical suspensions. Suspensions may be desirable, for example, for treatment of patients who are unable to swallow capsule or tablet dosage forms. Also, suspensions may facilitate treatment of gastrointestinal disorders, providing rapid and even dispersion of the pharmaceutical active in gastric fluids.

### Summary of the Invention

The present invention provides nitrofurantoin particulates being larger than 200 mesh size (74»m). The BET surface area of said particulates is at least 0.2 m²/g and the surface of said particulates consists essentially of nitrofurantoin monohydrate crystalline forms. Also preferably, said particulates comprise at least 5%, more preferably at least 50% of nitrofurantoin monohydrate. Preferred compositions of the invention comprise these nitrofurantoin particulates in an aqueous suspension.

The nitrofurantoin particulates of this invention are highly efficacious for the delivery of nitrofurantoin in oral dosage forms. In particular, this invention provides stable nitrofurantoin particulates useful in the formulation of stable, efficacious aqueous suspensions, for the treatment of gastrointestinal disorders and urinary tract infections, without undue side effects.

### DESCRIPTION OF THE DRAWINGS

The attached drawings are photomicrographs of different particulate forms of nitrofurantoin. Specifically:
Figure 1 depicts a perspective view of several nitrofurantoin particulates of this invention;
Figure 2 depicts a perspective view of several particles of macrocrystalline nitrofurantoin;
Figure 3 depicts a perspective view of several particles of nitrofurantoin monohydrate;
Figure 4 is a longitudinal sectional view of a portion of a nitrofurantoin particulate of this invention, comprising approximately 98% nitrofurantoin monohydrate;
Figure 5 is a longitudinal sectional view in perspective of a nitrofurantoin particulate of this invention, comprising approximately 67% nitrofurantoin monohydrate;
Figure 6 is a longitudinal sectional view of a portion of a nitrofurantoin particulate of this invention, comprising approximately 42% nitrofurantoin monohydrate; and
Figure 7 is a longitudinal sectional view in perspective of a nitrofurantoin particulate of this invention, comprising approximately 10% nitrofurantoin monohydrate.
A more detailed description of the drawings is set forth in the "Nitrofurantoin Particulates" subsection of the Description of the Invention, below.

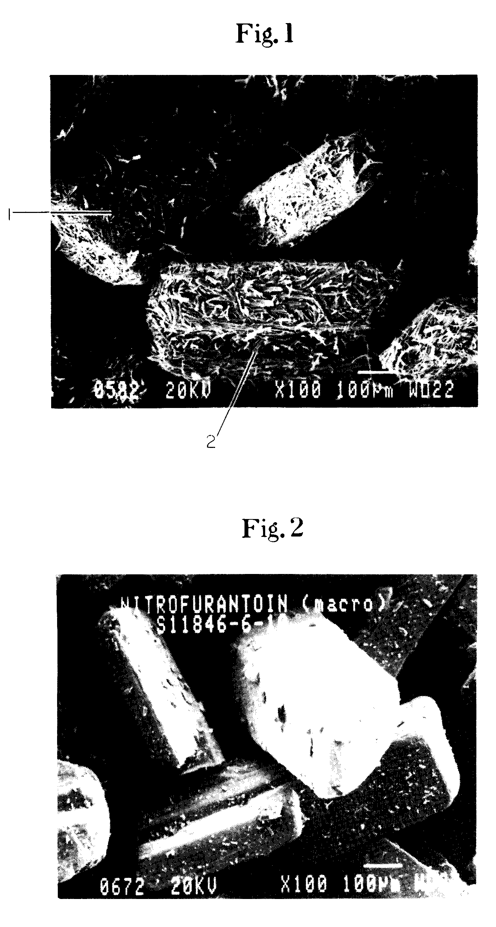

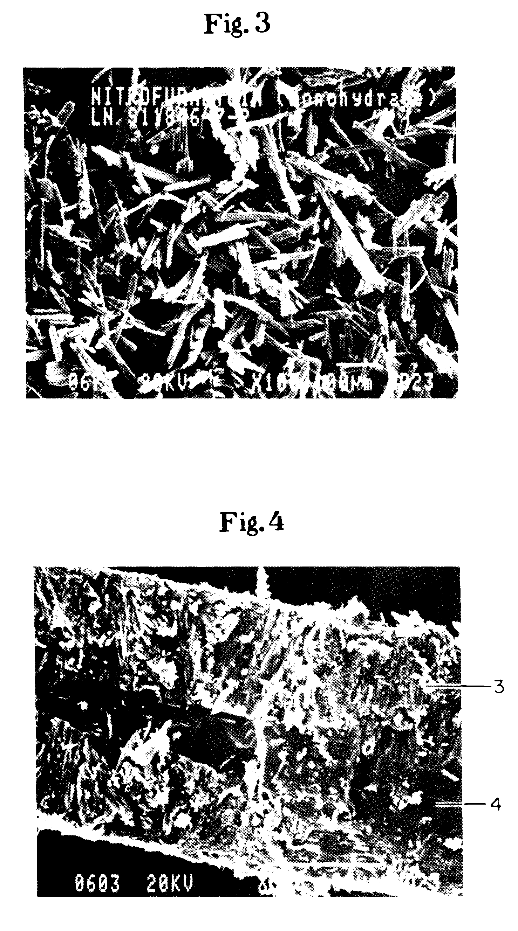

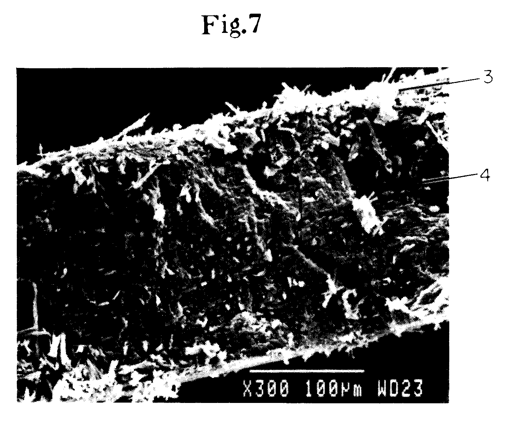

These photomicrographs were obtained by mounting the particulates on a Cambridge-type pin stub with double stick tape. (Sectional views were obtained by fracturing the particulates using gentle pressure from a glass coverslip.) The particulates, after mounting, were coated with 200A gold-palladium in a Balzers SCD 040 sputter coater. Examination of the crystals was in a JEOL 840 II scanning electron microscope operated at 20 KV (kilovolts). Images were recorded on a Polaroid P/N 55 film at a magnification of 100 for Figures 1-3, and 300 for Figures 4-7.

### DESCRIPTION OF THE INVENTION

The present invention encompasses certain novel nitrofurantoin particulates, methods for their manufacture, dosage forms, and methods of administering the nitrofurantoin particulates to a human or other animal subject. Specific compounds and compositions to be used in this invention must, accordingly, be pharmaceutically acceptable. As used herein, such a "pharmaceutically-acceptable" component is one that is suitable for use with humans/and or animals without undue adverse side effects (such as toxicity, irritation, and allergic response) commensurate with a reasonable benefit/risk ratio.

### Nitrofurantoin particulates:

"Nitrofurantoin" includes 1-[[(5-nitro-2-furanyl)methylene]-amino]-2,2-imidazolidinedione, and related compounds described in U.S. Patent 2,610,181, Hayes, issued September 9, 1952. Nitrofurantoin is described in the U.S. Pharmacopeia XXI. Nitrofurantoin may be made in a variety of physical forms, including (for example) "nitrofurantoin monohydrate" which contains about one mole of water chemically associated with one mole of nitrofurantoin. Nitrofurantoin monohydrate typically occurs in small, needlelike prisms. The crystals are transparent yellow with an adamantine luster. Such crystals are seen in Figure 3 of the drawings. "Anhydrous nitrofurantoin" contains essentially no chemically-bonded water, and typically occurs in pinacoid prisms with a length: width ratio of 3:1. The crystals are transparent to translucent yellow, with a nonmetallic waxy luster. Such crystals are seen in Figure 2 of the drawings. (Descriptive terms for crystals and particulates, used herein, are discussed in B. Mason and L. Berry, Elements of Mineralogy (1969), incorporated by reference herein.)

The "nitrofurantoin particulates" of this invention are comprised of discrete particulates of nitrofurantoin, wherein:
(a) said particulates are larger than 200 mesh size; (74 »m) and
(b) the BET surface area of said particulates is at least 0.2 m²/g (square meters per gram).
Preferably the particulates have a size distribution of from 30 mesh [590 »m] to 100 mesh [149 »m], more preferably from 40 mesh [420 »m] to 60 mesh [250 »m]. Preferably, the BET surface area of said particulates is at least 0.4 m²/g.

In the commercial manufacture of the nitrofurantoin particulates of this invention, the particle size and BET surface area of the particulates may vary somewhat from the ranges described herein. Such commercial materials may have a distribution of values for these parameters, with mean values within the ranges described above.

As referred to herein, "size" of the nitrofurantoin particulates of this invention refers to the measurement of the largest U. S. Standard mesh screen through which substantially all of the particulates will pass. In general, this mesh measurement is a function of the smallest dimension of the particulates being measured. As this minimum dimension of the particulates increases, the mesh size will decrease. For example, as used herein, particulates "larger than 200 mesh [74 »m] size" means that substantially all of the particulates will pass through a U. S. Standard mesh screen of less than 200 mesh [74 »m], with few particulates passing through the 200 mesh [74 »m] screen. Also, particulates having a size distribution "of from 40 mesh [420 »m] to 60 mesh [250 »m]" (also referred to as "40/60 mesh" particles) means that substantially all of the particulates will pass through a 40 mesh screen [420 »m], and essentially none of the particles will pass through a 60 mesh [250 »m] screen. Mesh measurements are discussed in "Screening", Chemical Engineer's Handbook, 4th Edition 21-46 (J. Perry, editor).

As referred to herein, "the BET surface area" of the nitrofurantoin particulates of this invention refers to the measurement of the surface area in immediate contact with an inert gas into which the particulate is placed, measured by the means generally described in S. Brunnaer et al., 60 J. American Chemical Society 309A (1938), using a single point measurement technique on a Micromeritics Flowsorb II 2300 (manufactured by Micromeritics Instrument Corporation, Norcross, Georgia, U.S.A.). The particulate sample is degassed at approximately 140°C (284°F) for approximately 15 minutes. The analysis gas is nitrogen/helium at a molar ratio of 30/70. Cooling baths are liquid nitrogen, and the apparatus is calibrated using nitrogen gas injected with a gas-tight syringe.

The nitrofurantoin particulates of this invention have bulk physical characteristics similar to those of macrocrystalline anhydrous nitrofurantoin. In particular, the nitrofurantoin particulates are typically pinacoid prisms with a length:width ratio of about 3:1. These particulates are acicular microcrystalline aggregates with a nonmetallic silky to earthy luster. Several of these nitrofurantoin particulates are depicted in Figure 1. Particulate 1 is a perspective view, showing both a face along its long axis and a face along its short axis. Particulate 2 is a perspective view, showing two faces along the long axis of the particulate. The similarity of the bulk physical characteristics of these particulates to the bulk physical characteristics of macrocrystalline anhydrous nitrofurantoin can be seen by comparison of the particulates depicted in Figure 1 with those depicted in Figure 2.

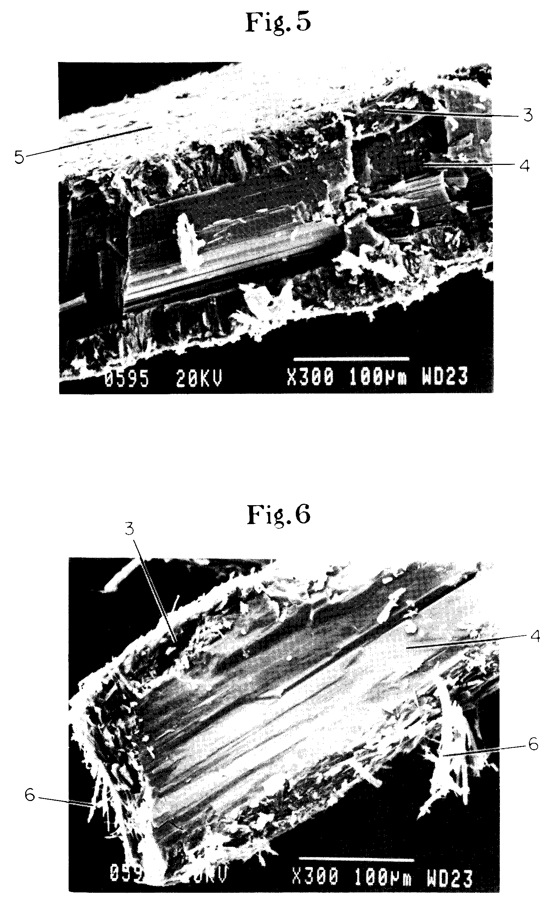

The surface of the nitrofurantoin particulates preferably consists essentially of nitrofurantoin monohydrate crystalline forms. The "surface" is the portion of the nitrofurantoin particulate that is in immediate contact with a fluid into which the particulate is submersed. The remaining, inner portion of the nitrofurantoin particulate (the "core") is comprised of anhydrous nitrofurantoin, nitrofurantoin monohydrate, or mixtures thereof. Figure 5, for example, depicts the surface 5 of a particulate, as well as the core 4 of the particulate. As used herein "nitrofurantoin monohydrate crystalline forms" are agglomerated nitrofurantoin particles, on the surface of the nitrofurantoin particulates of this invention, having a physical form substantially similar to nitrofurantoin monohydrate. The presence of such crystalline forms may be determined by scanning electron microscopy, using methods as described above. Said crystalline forms may be comprised of nitrofurantoin monohydrate or anhydrous nitrofurantoin. Such crystalline forms comprised of anhydrous nitrofurantoin readily rehydrate to form nitrofurantoin monohydrate when exposed to water; i.e. when approximately 1 gram of the particulates of this invention is placed in an open dish in a humidity chamber (as a thin layer of approximately 5 millimeters depth), for approximately 8 hours at approximately 44% relative humidity and 21°C (70°F). Preferably, the surface of the nitrofurantoin particulates of this invention consists essentially of nitrofurantoin monohydrate crystalline forms comprising nitrofurantoin monohydrate.

Accordingly, the nitrofurantoin particulates of this invention are preferably comprised of from 5% to 100% of nitrofurantoin monohydrate. More preferably, the nitrofurantoin particulates comprise at least 50%, more preferably at least 90%, nitrofurantoin monohydrate. Nitrofurantoin particulates containing less than 100% nitrofurantoin monohydrate consist of: a "surface layer" comprising nitrofurantoin monohydrate as the monohydrate crystalline forms on the surface, and in the contiguous portion of the core; with the remaining portion (if any) of the core comprising anhydrous nitrofurantoin.

The portion of nitrofurantoin particulates comprised by nitrofurantoin monohydrate and nitrofurantoin anhydrous may be determined using standard analytical techniques well known in the art. In particular, the relative proportion of nitrofurantoin monohydrate in the nitrofurantoin particulates may be determined by thermogravimetric analysis. The chemically-bonded water of the monohydrate is driven off by heating, resulting in a weight loss. This weight loss is a function of the amount of monohydrate in a sample analyzed. Specifically, a thermogravimetric scan is performed on a sample of nitrofurantoin particulates (unground) of from 5 to 10 milligrams, under nitrogen, over the temperature range of from 30°C (86°F) to 220°C (428°F), scanned at a rate of about 5°C per minute. The chemically-bound water is measured as the weight loss occurring in the range of from 80°C (176°F) to 150°C (302°F). This lost weight constitutes approximately 7% of the weight of the nitrofurantoin monohydrate originally in the sample. Accordingly, the weight of nitrofurantoin monohydrate in the sample is determined by multiplying the weight of lost water by approximately 14.3. This weight is then compared to the weight of the original sample, to determine the percentage of nitrofurantoin monohydrate in the sample. A general description of such analyses useful herein is set forth in Thermal Analysis, 3d edition (W. Wendlandt, editor, 1986)

The presence of nitrofurantoin monohydrate on the surface of the particulates may be determined by attenuated total reflectance infrared spectroscopy. Samples of particulates are placed on a KRS-5 crystal (having approximately 50 cm x 3 cm x 3 cm dimensions), at a 45 degree entry angle. A 4 cm⁻¹ resolution spectrum is obtained over the range of from 4000 cm⁻¹ to 450 cm⁻¹ . Distinct absorbance is seen at the following wavenumbers (+/- 5): 3618, 3474, 1778, 1723, 1132, 1018, 893, and 877. By comparison, absorbance for anhydrous nitrofurantoin is seen at the following wavenumbers (+/- 5): 1800, 1775, 1741, 1724, 1104, 1013, 901, and 867. Anhydrous nitrofurantoin will also pass identity test part B for nitrofurantoin, U.S. Pharmacopeia XXI, page 735.

### Methods of manufacture:

The nitrofurantoin particulates of this invention may be made by the method comprising the steps of:
(a) preparing a saturated aqueous solution of nitrofurantoin monohydrate;
(b) adding to said solution anhydrous nitrofurantoin having a particle size larger than 200 mesh (74 »m);
(c) mixing said solution for at least 5 minutes; and
(d) filtering said solution.
The anhydrous nitrofurantoin added in step (b) preferably has a particle size distribution of from 30 mesh [590 »m] to 100 [149 »m] mesh, more preferably from 40 mesh [420 »m] to 60 mesh [250 »m]. This particle size is selected to yield the particular particle size desired in the final nitrofurantoin particulate product. Preferably, said solution is mixed in step (c) for at least 6.5 hours. Also, preferably the anhydrous nitrofurantoin is added to the solution at a level of 100 grams/liter.

The anhydrous nitrofurantoin and the saturated nitrofurantoin monohydrate solution are mixed, in step (c), for a period of time sufficient to yield a particulate having a desired nitrofurantoin monohydrate composition. Nitrofurantoin particulates having low levels of nitrofurantoin monohydrate (i.e., 5%) are prepared by processes wherein this mixing step is 5 minutes. Nitrofurantoin particulates comprising 100% nitrofurantoin monohydrate are made by processes wherein the mixing step is continued for 24 hours. The specific duration of the mixing step will vary according to such factors as the size and configuration of the mixing vessel, the rate of mixing, and the size and surface characteristics of the nitrofurantoin anhydrous crystals used (the effective surface area of nitrofurantoin anhydrous exposed to the saturated solution of nitrofurantoin monohydrate). The specific mixing time to yield a nitrofurantoin particulate having a specific composition of nitrofurantoin monohydrate may be determined by routine experimentation.

### Compositions:

The compositions of this invention comprise:
(a) a safe and effective amount of nitrofurantoin particulates; and
(b) a pharmaceutically-acceptable carrier.
A "safe and effective amount" of nitrofurantoin particulates is an amount that is effective to inhibit microbial growth at the site of an infection to be treated in a human or lower animal subject, without undue adverse side effects (such as toxicity, irritation, or allergic response), commensurate with a reasonable benefit/risk ratio when used in the manner of this invention. The specific "safe and effective amount" will, obviously, vary with such factors as the particular condition being treated, the physical condition of the patient, the duration of treatment, the nature of concurrent therapy (if any), the specific dosage form to be used, the carrier employed, the solubility of the nitrofurantoin particulates therein, and the dosage regimen desired for the composition.

The compositions of this invention may be provided in unit dosage form. As used herein, a "unit dosage form" is a composition of this invention containing an amount of nitrofurantoin particulates that is suitable for administration to a human or lower animal subject, in a single dose, according to good medical practice. These compositions preferably contain from 5 mg (milligrams) to 1000 mg, more preferably from 10 mg to 200 mg, more preferably from 25 mg to 100 mg, of the nitrofurantoin particulates.

The compositions of this invention may be in any of a variety of dosage forms. Depending upon the particular rate of administration desired, a variety of pharmaceutically-acceptable carriers well known in the art may be used. These include solid or liquid fillers, diluents, hydrotropes, surface-active agents, and encapsulating substances. Optional pharmaceutically-active materials may be included, which do not substantially interfere with the antimicrobial activity of the nitrofurantoin particulates. The amount of carrier employed in conjunction with the nitrofurantoin particulates is sufficient to provide a practical quantity of material for administration per unit dose of the nitrofurantoin particulates Techniques and compositions for making dosage forms useful in the methods of this invention are described in the following references, 7 Modern Pharmaceutics, Chapters 9 and 10 (Banker & Rhodes, editors, 1979); Lieberman et al., Pharmaceutical Dosage Forms: Tablets (1981); and Ansel, Introduction to Pharmaceutical Dosage Forms 2d Edition (1976).

Various oral dosage forms can be used, including such solid forms as tablets, capsules, granules and bulk powders. These oral forms comprise a safe and effective amount, usually at least 5%, and preferably from 25% to 50%, of the nitrofurantoin particulates. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

Preferred compositions of this invention employ a suspension system comprising one or more compounds (herein "suspension agents") that maintain the nitrofurantoin particulates in an essentially uniform aqueous suspension at typical conditions of storage and use. Such suspension systems, suspension agents, and methods of their use include those well known in the art. See, for example, M. Pernarowski, "Solutions, Emulsions and Suspensions", Remington's Pharmaceutical Sciences (A. Osol, editor, 15th Edition, 1975). Suspension agents useful in the compositions of this invention include, for example, cellulose ethers (such as methylcellulose, hydroxyethylcellulose, and carboxymethylcellulose), alginates, carboxyvinylpolymers, xanthan gum, colloidal silicas, montmorillonite clays and hydrophobically treated montmorillonite clays (such as magnesium aluminum silicate), and mixtures thereof. Preferred suspension agents include mixtures of cellulose ethers and magnesium aluminum silicate.

One preferred suspension system employs a mixture of methylcellulose and magnesium aluminum silicate. In such a system, methylcellulose may be used at levels of from 0.1% to 10%, preferably from 0.5% to 1.5%, and magnesium aluminum silicate may be used at levels of from 0.19% to 10%, preferably from 2.5% to 4.0%. Methylcellulose, or cellulose methyl ether, is commercially available from a variety of sources as a chemically treated plant cellulose derivative. Among such methylcellulose materials useful herein is Methocel, sold by Dow Chemical Company. Magnesium aluminum silicate (or aluminum magnesium silicate) is of the formula Al₂MgO₈Si₂, occurring naturally in such smectite minerals as colerainite, saponite, and sapphirine. Refined magnesium aluminum silicates useful herein are readily available, such as Veegum, manufactured by R. T. Vanderbilt Company, Inc.

The compositions of this invention preferably contain an "antiseptic agent", i.e., one or more materials that prevent or arrest the growth or action of microorganisms by inhibiting their activity and/or by destroying them. These materials are preferably present at a level of from 0.001% to 0.5%. Many antiseptic materials are known in the art, including preservatives, disinfectants and antiseptics. Such materials are described, for example, in Disinfection, Sterilization and Preservation 3d (S. Block ed., 1983).

The compositions of the present invention may also contain optional components that modify the physical characteristics and/or therapeutic effects of the compositions. Such optional components must not, however, substantially affect, in an adverse manner, the therapeutic activity of the nitrofurantoin particulates. The optional components useful herein must not also substantially affect, in an undesired manner, the viscosity of the aqueous suspension. Preferred optional components useful herein include colorants, sweeteners, and flavorants, typically at levels of from 0.01% to 0.2%.

The pH of liquid suspensions of this invention is preferably from 1 to 6, more preferably from 3.5 to 5.0. The pH may be adjusted by addition of a pharmaceutically-acceptable acid or base. Suitable acids include, for example, hydrochloric acid and carboxylic acids such as citric acid, tartaric acid, and succinic acid. Suitable bases include, for example, the oxides and hydroxides of calcium, potassium, sodium and magnesium, alkaline quaternary compounds, alkaline amino acids, and mixtures thereof.

The compositions of this invention may be made by any of a variety of processes well known in the industry. Such processes typically involve admixture of the components, followed by homogenizing. As will be appreciated by those skilled in the art, the conditions under which the compositions are mixed and homogenized may have an effect on the product viscosity.

An infectious disorder in a human or other animal subject can be treated or prevented by administering a safe and effective amount of nitrofurantoin particulates to said subject. As used herein an "infectious disorder" is any disorder characterised by the presence of a microbial infection. A composition comprising the nitrofurantoin particulates of the present invention is preferably used for the treatment of bacterial infections, particularly for genito-urinary infections, and gastrointestinal infections.

The specific dosage of nitrofurantoin particulates to be administered, as well as the duration of treatment, are mutually dependent. The dosage and treatment regimen will also depend upon such factors as the route of administration, the type of dosage form used, the infectious agent present, the ability of the nitrofurantoin particulates to reach sustained effective levels at the site of the infection, the nature and extent of other infections (if any), the personal attributes of the subject (such as weight), compliance with the treatment regimen, and the presence and severity of any side effects of the treatment.

Typically, for a human adult (weighing approximately 70 kilograms), from 1 mg to 1,000 mg, more preferably from 10 mg to 400 mg, more preferably from 20 mg to 200 mg, of nitrofurantoin particulates are administered per day. Treatment regimens preferably extend from 3 to 56 days, preferably from 7 to 28 days, in duration.

A composition comprising the nitrofurantoin particulates of the present invention is preferably used for the treatment and prophylaxis of upper-gastrointestinal disorders mediated by Campylobacter pylori, (see EP-A-219,912, Kraff et al., published April 29, 1987). Nitrofurantoin particulates according to the present invention for the treatment of a human or other animal subject having such upper-gastrointestinal disorders are administered to said subject at a level of from 10 mg to 400 mg per day, for from 3 to 60 days.

### EXAMPLE I

Nitrofurantoin particulates of this invention are made by adding approximately 200 g (grams) of nitrofurantoin monohydrate to approximately 40 L (liters) of deionized water, in a suitable container. This mixture is stirred for approximately 30 minutes, at ambient temperature (approximately 68°F, 20°C). The mixture is then filtered, removing the undissolved nitrofurantoin monohydrate.

Approximately 4 kg (kilograms) of 40/60 mesh screened anhydrous nitrofurantoin macrocrystals are then added to the saturated nitrofurantoin monohydrate solution. This mixture is then stirred for approximately 6.5 hours, at ambient temperature.

The mixture is then filtered, and the nitrofurantoin particulates washed with ether. The particulates are then air dried for approximately 1 hour. The particulates are further dried for approximately 24 hours at 60°C (140°F).

Thermal gravimetric analysis of the particulates indicates that the particulates are comprised of approximately 98% nitrofurantoin monohydrate. The particles are screened, and are found to have a size of from 40 [420 »m] to 60 mesh [250 »m]. The BET surface area is measured to be approximately 5.8 m²/g. An attenuated reflectance infrared spectrum is performed, and distinct absorbance is seen at the wavenumbers characteristic of nitrofurantoin monohydrate.

Capsule dosage forms, according to this invention, are made by filling gelatin capsules, with approximately 100 mg of the nitrofurantoin particulates in each shell. A human subject suffering from a urinary tract infection caused by Escherichia coli is then administered one of these capsules, four times a day, for seven days. The infection is eradicated.

### EXAMPLE II

A composition of this invention is made having the following composition:

| Component | % (by weight) |
|---|---|
| nitrofurantoin particulates* | 0.508 |
| nitrofurantoin monohydrate | 0.018 |
| magnesium aluminum silicate | 3.010 |
| sodium carboxymethylcellulose | 1.180 |
| glycerin | 13.570 |
| sorbitol | 15.050 |
| methyl paraben | 0.129 |
| propyl paraben | 0.022 |
| citric acid | 0.726 |
| purified water | 65.787 |

| | |
|---|---|
| * made according to Example I | |

The magnesium aluminum silicate is added to approximately one third of the water, and mixed for approximately one hour, at approximately 50°C (122°F). Separately, the glycerin, methyl paraben, propyl paraben, and carboxymethylcellulose are mixed. This mixture is then slowly added to the magnesium aluminum silicate/water mixture. The sorbitol, citric acid and remaining water is added, and the mixture stirred for approximately 1.5 hours.

The nitrofurantoin monohydrate is then added to the solution, and mixed for approximately 2 hours. The nitrofurantoin particulates are added, and the mixture stirred for approximately 3 hours.

A human subject suffering from gastritis mediated by Campylobacter pylori is administered approximately 20 ml of this suspension (approximately 100 mg of nitrofurantoin), 4 times a day, for 28 days. Stomach cultures of the subject indicate the organism has been eradicated, with corresponding improvement in the subject's symptoms.

## Claims

1. Nitrofurantoin particulates being larger than 74 »m (200 mesh size) characterized in that the BET surface area of said particulates is at least 0.2 m²/g and that the surface of said particulates consists essentially of nitrofurantoin monohydrate crystalline forms.

2. Nitrofurantoin particulates, according to Claim 1, wherein said BET surface area is at least 0.4 m²/g.

3. Nitrofurantoin particulates, according to Claim 2, wherein said particulates have a size distribution of from 250 »m to 420 »m (40 mesh to 60 mesh).

4. Nitrofurantoin particulates, according to any of the preceding claims, wherein said particulates are comprised of at least 50% nitrofurantoin monohydrate.

5. Nitrofurantoin particulates, according to Claim 4 comprising at least 90% nitrofurantoin monohydrate.

6. A method of making nitrofurantoin particulates, wherein said particulates are larger than 74 »m (200 mesh size), and the BET surface area of said particulates is at least 0.2 m/g and the surface of said particulates consists essentially of nitrofurantoin monohydrate crystalline forms, comprising the steps of:
(a) preparing a saturated aqueous solution of nitrofurantoin monohydrate;
(b) adding to said solution anhydrous nitrofurantoin having a particle size larger than 74 »m(200 mesh size);
(c) mixing said solution for at least 5 minutes; and
(d) filtering said solution.

7. A method of making nitrofurantoin particulates, according to Claim 6, wherein said anhydrous nitrofurantoin has a particle size distribution of from 250 »m to 420 »m (40 mesh to 60 mesh).

8. A method of making nitrofurantoin particulates, according to Claim 7, wherein said anhydrous nitrofurantoin is added to said solution at a level of 100g per liter of said solution.

9. A method of making nitrofurantoin particulates, according to Claim 6, wherein said solution is mixed in said step (c) for at least 6.5 hours.

## Patentansprüche

1. Teilchenförmiges Nitrofurantoin, dessen Teilchen größer als 74 »m (mesh-Größe 200) sind, dadurch gekennzeichnet, daß die BET-Oberfläche dieses teilchenförmigen Materials wenigstens 0,2 m²/g beträgt und daß die Oberfläche des teilchenförmigen Materials im wesentlichen aus kristallinen Formen von Nitrofurantoinmonohydrat besteht.

2. Teilchenförmiges Nitrofurantoin nach Anspruch 1, wobei die BET-Oberfläche wenigstens 0,4 m²/g beträgt.

3. Teilchenförmiges Nitrofurantoin nach Anspruch 2, wobei das teilchenförmige Material eine Teilchengrößenverteilung von 250 »m bis 420 »m (40 mesh bis 60 mesh) aufweist.

4. Teilchenförmiges Nitrofurantoin nach einem der vorhergehenden Ansprüche, wobei das teilchenförmige Material wenigstens zu 50 % aus Nitrofurantoinmonohydrat besteht.

5. Teilchenförmiges Nitrofurantoin nach Anspruch 4, welches wenigstens 90 % Nitrofurantoinmonohydrat enthält.

6. Verfahren zur Herstellung von teilchenförmigem Nitrofurantoin, dessen Teilchen größer als 74 »m (mesh-Größe 200) sind, wobei die BET-Oberfläche dieses teilchenförmigen Materials wenigstens 0,2 m²/g beträgt und wobei die Oberfläche des teilchenförmigen Materials im wesentlichen aus kristallinen Formen von Nitrofurantoinmonohydrat besteht, welches Verfahren die folgenden Stufen umfaßt:
(a) Herstellen einer gesättigten wäßrigen Lösung von Nitrofurantoinmonohydrat;
(b) Hinzufügen von wasserfreiem Nitrofurantoin mit einer Teilchengröße größer als 74 »m (200 mesh) zu dieser Lösung;
(c) Vermischen dieser Lösung während wenigstens 5 min; und
(d) Filtrieren dieser Lösung.

7. Verfahren zur Herstellung von teilchenförmigem Nitrofurantoin nach Anspruch 6, wobei das wasserfreie Nitrofurantoin eine Teilchengrößenverteilung von 250 »m bis 420 »m (40 mesh bis 60 mesh) aufweist.

8. Verfahren zur Herstellung von teilchenförmigem Nitrofurantoin nach Anspruch 7, wobei das wasserfreie Nitrofurantoin zu der Lösung in einer Menge von 100 g/l der Lösung zugesetzt wird.

9. Verfahren zur Herstellung von teilchenförmigem Nitrofurantoin nach Anspruch 6, wobei die Lösung in der Stufe (c) während wenigstens 6,5 h vermischt wird.

## Revendications

1. Particules de nitrofurantoïne, de granulométrie supérieure à 74 »m (200 mesh), caractérisées en ce que la surface spécifique BET desdites particules est d'au moins 0,2 m²/g et en ce que la surface desdites particules est essentiellement formée de cristaux de monohydrate de nitrofurantoïne.

2. Particules de nitrofurantoïne selon la revendication 1, dans lesquelles ladite surface spécifique BET est d'au moins 0,4 m²/g.

3. Particules de nitrofurantoïne selon la revendication 2, dans lesquelles lesdites particules ont une distribution granulométrique de 250 »m à 420 »m (40 mesh à 60 mesh).

4. Particules de nitrofurantoïne selon l'une quelconque des revendications précédentes, dans lesquelles lesdites particules sont constituées d'au moins 50% de monohydrate de nitrofurantoïne.

5. Particules de nitrofurantoïne selon la revendication 4, comprenant au moins 90% de monohydrate de nitrofurantoïne.

6. Procédé de fabrication de particules de nitrofurantoïne, dans lequel lesdites particules ont une granulométrie supérieure à 74 »m (200 mesh), et la surface spécifique BET desdites particules est d'au moins 0,2 m²/g et la surface desdites particules est essentiellement formée de cristaux de monohydrate de nitrofurantoïne, comprenant les étapes qui consistent à:
(a) préparer une solution aqueuse saturée de monohydrate de nitrofurantoïne;
(b) ajouter à ladite solution de la nitrofurantoïne anhydre ayant une granulométrie supérieure à 74 »m (200 mesh);
(c) mélanger ladite solution pendant au moins 5 minutes; et
(d) filtrer ladite solution.

7. Procédé de fabrication de particules de nitrofurantoïne selon la revendication 6, dans lequel ladite nitrofurantoïne anhydre a une distribution granulométrique de 250 »m à 420 »m (40 à 60 mesh).

8. Procédé de fabrication de particules de nitrofurantoïne selon la revendication 7, dans lequel ladite nitrofurantoïne anhydre est ajoutée à ladite solution à raison de 100 g par litre de ladite solution.

9. Procédé de fabrication de particules de nitrofurantoïne selon la revendication 6, dans lequel ladite solution est mélangée dans ladite étape (c) pendant au moins 6,5 heures.
